# EUROPEAN PATENT APPLICATION

(11) **EP 2 298 330 A1**
(43) Date of publication of application: **23.03.2011**
(21) Application number: 09011884.5
(22) Date of filing: 17.09.2009
(51) Int. Cl.: A61K 36/75

(54) **Iron-complexes extracted from curry leaves and their use**

(71) Applicant: Biogena Naturprodukte GmbH & Co KG, 5020 Salzburg (AT)
(72) Inventor: Schmidbauer, Albert, 5020 Salzburg (AT); Leisser, Christina Dr., 1070 Wien (AT)
(74) Representative: Peters, Hajo

(57) **Abstract**

The present invention refers to compositions extracted from leaves of the curry tree (*Murraya koenigii*) comprising iron, medicaments comprising such compositions, the use of such compositions for the production of a medicament for the treatment of iron deficiency conditions or as a dietary supplement.

## Description

### Field of the invention

The present invention refers to compositions extracted from leaves of the curry tree (*Murraya koenigii*) comprising iron, medicaments comprising such compositions, the use of such compositions for the production of a medicament for the treatment of iron deficiency conditions or as a dietary supplement.

### Background of the invention

According to the US Department of Health and Human Services iron deficiency is the most common nutritional deficiency and the leading cause of anemia in the United States. Worldwide it is assumed that approximately 1 billion people are suffering from iron deficiency. According to above source, iron deficiency can cause fatigue that impairs the ability to do physical work in adults and it may also affect memory or other mental functions. Further, iron deficiency can delay normal infant motor function or mental function, whereas anemia during pregnancy can increase the risk for small or early (preterm) babies. Iron deficiency might result either from increased iron needs like present e.g. in pregnant women, in people losing blood like in women with heavy menstrual periods, or frequent blood donators, as well as in people with some stomach and intestinal conditions blocking iron uptake. Also infants and toddlers need more iron than older children, because of their rapid growth. On the other hand the amount of iron absorbed from diet might not be sufficient to avoid iron deficiency occurring.

More than 75% of women of child-bearing age consume less iron than recommended (Bundesforschungsinstitut für Ernährung und Lebensmittel: Nationale Verzehrsstudie 2008). Also during pregnancies, during strong menstruation, or in athletes or during gastro-intestinal bleedings or diseases a clinically relevant or sub-clinical deficiency might occur.

Iron deficiency will lead to a reduced concentration of hemeglobin and erythrocytes with unspecific symptoms finally leading to a form of anemia. The reduced transport of oxygen will lead to an impaired supply to organs and tissue leading to symptoms like tiredness and exhaustion and an increased susceptibility to infections. Iron deficiency will finally lead to a hypo chrome microcytic anemia shown by formation of small erythrocytes lacking haemoglobin.

Iron is abundantly present in food as a tracer element, but still its absorption into the body might be negatively influenced by many different factors. Thus, inorganic Fe(II) or Fe(III) compounds are often only badly absorbed as they tend to form badly soluble complexes in the mild basic environment of the upper ileum.

On the other hand iron, being bound to porphyrine rings as in hemoglobine, myoglobine or forms of cytochromes are opposed to that quite effectively absorbed. In plants iron is partly present in the structure of cytochrome b containig iron-protoporphyrine or as ferritin. Even though in principle iron compounds from plant sources will be absorbed in the same amount as iron compounds from animal sources the presence of chelating agents typical to plants like phytic acid, oxalic acid or tannins will impair bio availability.

There are a number of dietary supplements or pharmaceutical formulations providing additional iron to a person in need. This iron usually is supplied in form of a salt (like inorganic or organic salt) or an iron complex. The most common source of iron provided are oral formulations comprising iron in form of iron(II) sulphate, but also complexes with gluconate, dextran or fumarate.

These standard oral iron preparations usually containing iron salts just show an average bioavailability, which still often cause gastro-intestinal ailments (Mutschler E: Arzneimittelwirkungen WVG Stuttgart, 2001). Oral dietary supplements comprising iron salts clearly show a limited gastrointestinal tolerability. Approximately 20% of the users show side effects such as flatulence, pain, obstipation and nausea causing the withdrawal of 5% of the participants in these clinical studies (Martius F: Eisenmangel ohne Anämie; Curriculum Schweiz. Med. Forum 2009; 9 (15-16):294-299).

According to the website of the US Department of Health and Human Services mentioned above Iron from meat, poultry, and fish (i.e., heme iron) is absorbed two to three times more efficiently than iron from plants (i.e., non-heme iron). Thus, even though careful meal planning could help increase the amount of iron absorbed, a vegetarian diet tends to be low in heme iron. Still, for a number of reasons including religion, environmental or health consciousness or other ethical believes a considerable part of the population like e.g. vegetarians would dearly prefer iron as supplement or cure being acquired from a plant. In addition, even though heme iron is believed to be better utilized and tolerated than non-heme bound iron, the fact that heme iron is derived from animals is limiting its pharmaceutical usage as well as is its acceptance by patients and costumers even with non-vegetarians.

For all the above reasons there was a clear need to provide a source of iron useful as dietary supplement or medicament for treating iron deficiency or anemia in which the iron would be stemming from plants, especially a source of iron ensuring a high tolerability with the patient/person in need thereof as - especially in a dietary supplement - any side effect naturally also has a high effect on compliance.

This problem was solved by providing a composition extracted from the leaves of the curry tree (*murraya koenigii*) comprising at least 1 weight % of iron. Besides a higher bioavailability achieved with the iron derived from leaves of the curry tree if compared to iron salts like iron gluconate, also a dietary supplement with the iron derived according to the invention showed a much higher tolerability.

"Iron" according to this invention is defined as iron in any form, including its elemental form, salts or oxides or as iron complexes like being bound to porphyrine rings as in hemoglobine, myoglobine or forms of cytochromes. In principle the iron may be in ferrous (Fe(II)) or ferric Fe(III) form. Preferably, the iron according to this invention is in its Fe(II) (ferrous form) and/or is either in form of an Fe(II)-complex or not.

"Weight % of iron" according to this invention is defined as the content (in weight) of elemental iron expressed as percentage of the overall weight of a composition, medicament (also like the content of a capsule).

"X mg of iron" according to this invention is defined as the amount of elemental iron being X mg in the composition or medicament (like a capsule) or being applied to a subject.

In a further embodiment the Composition according to the invention comprises at least 2 weight % of iron, preferably at least 3 weight % of iron, more preferably at least 3.5 weight % of iron.

In a further embodiment the Composition according to the invention is comprising 1 to 8 weight % of iron, preferably 2 to 7 weight %, more preferably 3 to 6 weight %, most preferably 3.5 to 5.5 weight % of iron.

In a further embodiment of the Composition according to the invention the Composition is obtainable by a process comprising the following steps:
- Step (i): Optionally milling or grinding of the leaves of *murraya koenigii* to achieve a powder of the leaves before the extraction step (ii);
- Step (ii): Extracting leaves of *murraya koenigii* at least once with an aqueous solution;
- Step (iii): Optionally, filter the extract from step (ii);
- Step (iv): Optionally, at least partly remove solvent from the extract from step (ii) or (iii);
- Step (v): Remove solvent from the extract from step (ii), (iii) or (iv) until dryness;
- Step (vi): Powdering the dry product of step (v);
- Step (vii): Optionally sieving the powdered product of step (vi).

In a further embodiment of this Composition according to the invention obtainable by above process the following modifications to the steps might apply:
- Before step (i) the leaves of *murraya koenigii* are optionally pretreated by one or more optional operation such as washing, cleaning, soaking, drying, cutting, chopping, and/or blanching; and/or
- In step (i), the milling and grinding of the leaves of *murraya koenigii* to achieve a powder of the leaves before the extraction step (ii) is done with a mechanical device; and/or
- In step (ii):
   ➢ the extraction of the leaves of *murraya koenigii* is done with a solution comprising more than 50%(v/v) of water, preferably is water; and/or
   ➢ the extraction of the leaves of *murraya koenigii* is repeated at least once, preferably is repeated once, twice or thrice; and/or
   ➢ the extraction of the leaves of *murraya koenigii* is done at ambient temperature or above, preferably is done above 50°C, more preferably is done between 60 and 90°C, most preferably between 70 and 80°C.

A further aspect of the invention refers to a process for the production of a composition according to the invention, comprising the following steps:
- Step (i): Optionally milling or grinding of the leaves of *murraya koenigii* to achieve a powder of the leaves before the extraction step (ii);
- Step (ii): Extracting leaves of *murraya koenigii* at least once with an aqueous solution;
- Step (iii): Optionally, filter the extract from step (ii);
- Step (iv): Optionally, at least partly remove solvent from the extract from step (ii) or (iii);
- Step (v): Remove solvent from the extract from step (ii), (iii) or (iv) until dryness;
- Step (vi): Powdering the dry product of step (v);
- Step (vii): Optionally sieving the powdered product of step (vi).

In a further embodiment of this Process according to the invention the following modifications to the steps might apply:
- Before step (i) the leaves of *murraya koenigii* are optionally pretreated by one or more optional operation such as washing, cleaning, soaking, drying, cutting, chopping, and/or blanching; and/or
- In step (i), the milling and grinding of the leaves of *murraya koenigii* to achieve a powder of the leaves before the extraction step (ii) is done with a mechanical device; and/or
- In step (ii):
   ➢ the extraction of the leaves of *murraya koenigii* is done with a solution comprising more than 50%(v/v) of water, preferably is water; and/or
   ➢ the extraction of the leaves of *murraya koenigii* is repeated at least once, preferably is repeated once, twice or thrice; and/or
   ➢ the extraction of the leaves of *murraya koenigii* is done at ambient temperature or above, preferably is done above 50°C, more preferably is done between 60 and 90°C, most preferably between 70 and 80°C.

A further aspect of the invention refers to a medicament comprising a composition according to the invention and optionally one or more pharmaceutically acceptable excipients.

The medicament according to the present invention may be in any form suitable for the application to humans and/or animals, preferably humans including infants, children and adults and can be produced by standard procedures known to those skilled in the art. The composition of the medicament may vary depending on the route of administration, whereby oral administration is preferred.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The solid compositions may come in many forms including e.g. the form of tablets, capsules or pellets including capsules filled with pellets. The solid oral compositions (which are preferred) may be prepared by conventional methods of blending, filling or tabletting. Repeated blending operations may be used to distribute the Composition according to the invention throughout those medicaments employing large quantities of fillers. Such operations are conventional in the art. The tablets may for example be prepared by wet or dry granulation and optionally coated according to the methods well known in normal pharmaceutical practice, in particular with an enteric coating. Most preferably still, the composition according to the invention is filled into capsules.

The mentioned formulations will be prepared using standard methods such as those described in or referred to in Pharmacopeias and similar reference texts.

Liquid oral forms for administration may also contain certain additives such as sweeteners, flavouring, preservatives, and emulsifying agents. Non-aqueous liquid compositions for oral administration may also be formulated, containing edible oils. Such liquid compositions may be conveniently encapsulated in e.g. gelatine capsules in a unit dosage amount.

The daily dosage for humans and animals may vary depending on factors that have their basis in the respective species or other factors, such as age, sex, weight or degree of iron deficiency and so forth. The daily dosage for humans may preferably be in the range from 10 to 250 of elemental iron, preferably 20 to 200, or preferably 40 to 125 milligrams of elemental iron to be administered during one or several intakes per day. The uptake is adapted to the severity of the iron deficiency. The dosage per each dosage unit like a tablet or capsule might range between 10 to 50 mg, 15 to 40 mg or 20 to 30 mg of iron.

In a further embodiment in the Medicament according to the invention the iron comprised therein fully stems from plant sources, preferably from the composition according to the invention. This means that all or substantially all iron in the medicament (pharmaceutical formulation) is derived from plant origin, in a most preferred embodiment completely or nearly completely from the leaves of the curry tree (*murraya koenigii*) by being filled with the Composition according to the invention extracted from the leaves of the curry tree (*murraya koenigii*) comprising at least 1 weight % of iron.

In a further embodiment the Medicament according to the invention comprises at least 2 weight % of iron, preferably at least 3 weight % of iron, more preferably at least 3.5 weight % of iron.

In a further embodiment the Medicament according to the invention is comprising 1 to 8 weight % of iron, preferably 2 to 7 weight %, more preferably 3 to 6 weight %, most preferably 3.5 to 5.5 weight % of iron.

In a further embodiment the Medicament according to the invention comprises between 10 to 50 mg, 15 to 30 mg or 20 to 30 mg of iron per dosage unit like a tablet or capsule. Preferably all or substantially all of the iron are stemming/derived from the composition according to the invention. In one embodiment the Medicament according to the invention comprises 21 mg of iron per capsule; preferably with the iron (preferably all or substantially all of it) stemming/derived from the composition according to the invention.

In a further embodiment the Medicament according to the invention further comprises Vitamin C.

In a further embodiment the Medicament according to the invention comprises 1 to 8 weight-% of iron and optionally 2 to 5 weight-% of Vitamin C.

In a further embodiment the Medicament according to the invention the Vitamin C comprised therein fully stems from plant sources, preferably from acerola (*Malpighia glabra*) fruits. This means that all or substantially all Vitamin C in the medicament (pharmaceutical formulation) is derived from plant origin

In a further embodiment the Medicament according to the invention comprises between 1 to 1000 mg, 5 to 500 mg or 10 to 100 mg of vitamin C (ascorbic acid) per dosage unit like a tablet or capsule. Preferably all or substantially all of the vitamin C is stemming/derived from plant sources like acerola fruits. In one embodiment the Medicament according to the invention comprises 15 mg of vitamin C per capsule; preferably with the vitamin C (preferably all or substantially all of it) stemming/derived from plant sources like acerola fruits.

In a further embodiment the Medicament according to the invention comprises between 10 to 50 mg, 15 to 30 mg or 20 to 30 mg of iron and between 1 to 1000 mg, 5 to 500 mg or 10 to 100 mg of vitamin C (ascorbic acid) per dosage unit like a tablet or capsule. Preferably all or substantially all of the iron are stemming/derived from the composition according to the invention. Preferably all or substantially all of the vitamin C is stemming/derived from plant sources like acerola fruits. In one embodiment the Medicament according to the invention comprises 21 mg of iron and 15 mg of vitamin C per capsule; preferably with the iron (preferably all or substantially all of it) stemming/derived from the composition according to the invention and the vitamin C (preferably all or substantially all of it) stemming/derived from plant sources like acerola fruits.

A further aspect of the invention refers to the use of a composition according to the invention for the production of a medicament for the treatment of iron deficiency, iron deficiency conditions, anemia, or iron deficiency anemia.

A further aspect of the invention refers to a composition according to the invention for use as a dietary supplement.

A further aspect of the invention refers to a composition according to the invention for use in supplementing an increased need for iron during pregnancy or lactation, during adolescence or competitive sports, following strong blood loss after surgery, gastrointestinal bleedings or strong menstruation; or during impairment of digestion or absorption of iron or diarrhea, or gastro-intestinal disease.

A further embodiment of the invention is related to a method for treating iron deficiency conditions in a mammal by administering to a subject in need of said treatment a composition or medicament according to the invention. Preferably, this mammal is a human being, most preferably a woman, man or child. Further, the iron deficiency condition might be the result of blood loss like e.g. in women with heavy menstrual periods, or frequent blood donators, might occur in pregnant women, or in people with some stomach and intestinal conditions blocking iron uptake. Further, the iron deficiency condition might be the result of lack of iron absorbed from the diet. Further, if the iron deficiency condition is acute the composition according to the invention to be administered is administered so that the dosage of iron applied to the subject adds up to between 40 and 125 mg of iron.

The examples and figures in the following section describing pharmacological trials are merely illustrative and the invention cannot be considered in any way as being restricted to these applications.

### Figures:

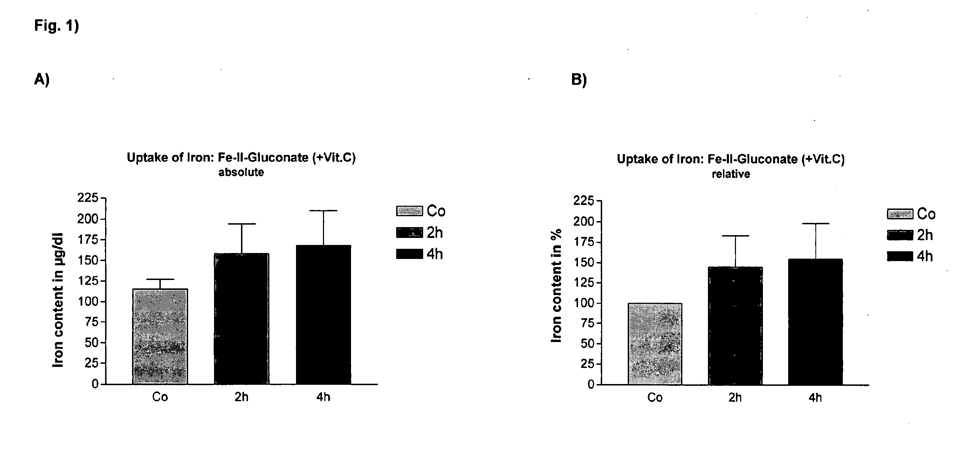

**Figure 1****)** refers to Example 7 and illustrates the amount of iron taken up over time when orally applying iron as Fe-II-gluconate salt with vitamin C (as known in the art) expressed as the absolute amount (A) or relative increase (B) of iron content in blood (serum) of the probands.

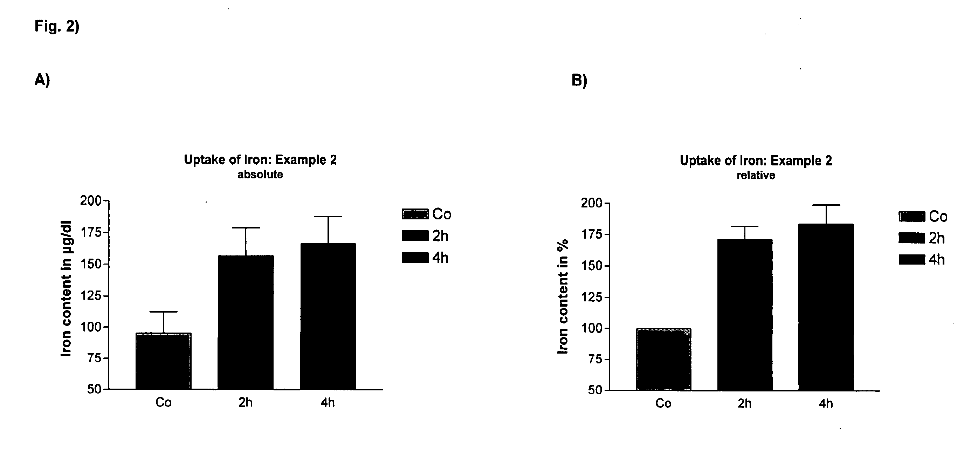

**Figure 2****)** refers to Example 7 and illustrates the amount of iron taken up over time when orally applying iron in the form of the composition according to the invention (example 2) expressed as the absolute amount (A) or relative increase (B) of iron content in blood (serum) of the probands.

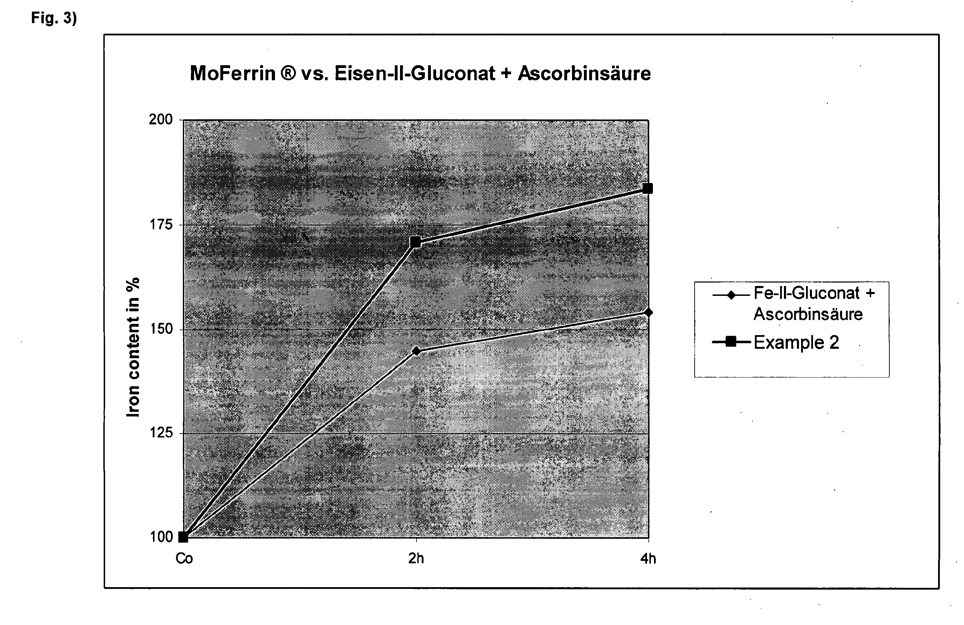

**Figure 3****)** refers to Example 7 and compares the amount of iron taken up over time when orally applying iron in the form of the composition according to the invention (example 2) or as Fe-II-gluconate salt with vitamin C (as known in the art) expressed as relative increase of iron content in blood (serum) of the probands.

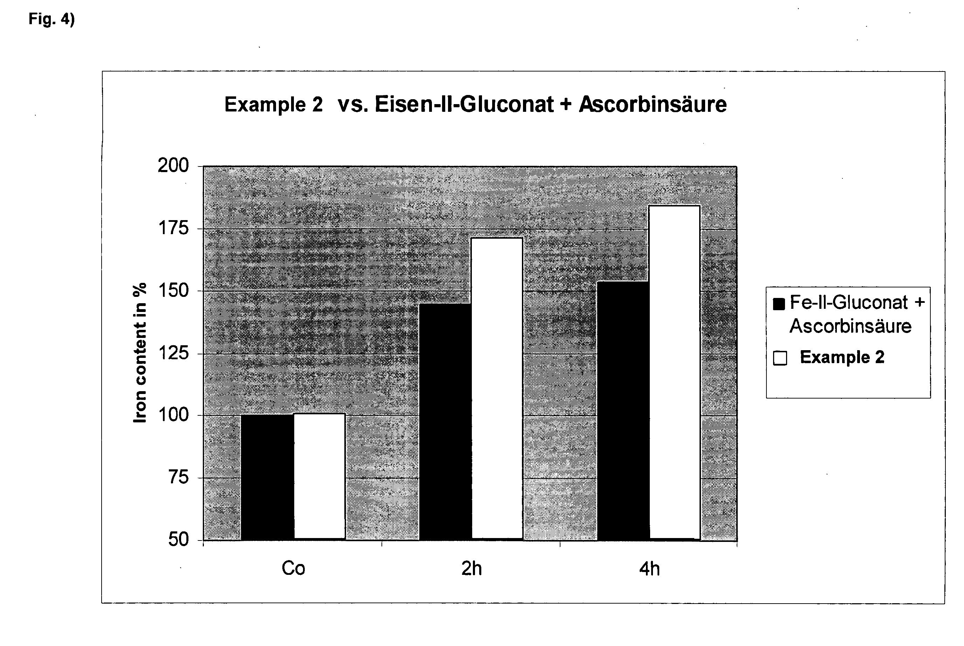

**Figure 4****)** refers to Example 7 and compares the amount of iron taken up over time when orally applying iron in the form of the composition according to the invention (example 2) or as Fe-II-gluconate salt with vitamin C (as known in the art) expressed as relative increase of iron content in blood (serum) of the probands.

### Examples

### Example 1: Extraction Process from Curry leaves

### Example 1A: General Extraction Process

1000 kg of powdered leaves from the curry tree (*Murraya koenigii*) were repeatedly extracted with water. The resulting watery extract was filtered and further concentrated under vacuum (removing part of the water). Thereafter, the concentrated extract was spray dried and powdered. Following that the powdered extract was sieved and blended. Finally the blend was sterilized by heat, sieved again and packaged (e.g. into 20 kg portions).

### Example 1B: Exemplary Extraction Process

Leaves of the curry tree (*Murraya koenigii*) are collected and dried in open-air under a shade. The dried material is ground in a swing hammer mill. In the extraction step, about 1000 kg of this dry powder material is charged to an extractor. The extractor includes a stainless steel vessel of about 5,000 L capacity provided with an agitator system and a surrounding jacket for steam heating. About 2,000 to 3,000 L of water as a solvent is charged into the extractor. The extractor contents are maintained at about 70-75° C. by heating with steam and the extraction is carried out for about six hours. This extract is referred to as "A". The volume of extract "A" is about 1,500 to 2,000 L.

The residue in the extractor comprising the partly-extracted curry tree plant material is subjected to a second extraction (leaching) operation. About 2,000 to 3,000 L of water is charged into the extractor and the extraction carried out at about 70-75° C. The extract is taken out of the extractor. The quantity of extract obtained was about 1,500 to 2,000 L. This extract is referred to as "B".

The plant residue in the extractor, comprising the twice-extracted curry tree plant material is optionally subjected to a third extraction. About 2,000 to 3,000 L of water was charged into the reactor(extractor) to yield about 1,500 L to 2,000 L of extract at the end of the extraction operation which was carried out at about 70-75° C. This extract is referred to as "C".

For a first filtration step, the extracts "A" and "B" and optionally "C" are - either separately or jointly - filtered. This filtering is done in a stainless steel Nutsche type Filter. The filter bed is washed with water. The filtrate contains the iron complexes.

For a concentration step, the filtrates "A" and "B" and optionally "C" are either separately or jointly concentrated in concentrators at about 40 to 60°C and under vacuum.

Thereafter, the concentrated extract was spray dried and powdered. The concentrated material constitutes the first Curry tree extract product. Following that the powdered extract was sieved and blended. Finally the blend was sterilized by heat, sieved again and packaged (e.g. into 20 kg portions).

### Example 2: Composition prepared according to example 1

The dried composition prepared according to example 1A comprised approximately 3 and 5 weight-% of iron. The difference was being due to the non-uniform nature of biological material. In a standard production, 500 mg of the composition resulted in 18 mg of natural iron being present (3.6 weight %) (= Standard Composition).

### Example 3: Pharmaceutical Formulation 1

583 mg of Standard Composition (see Example 2) were filled into a standard pharmaceutical capsule (most often designed for 1 g of material) made from hydroxypropylmethylcellulose. The hydroxypropylmethylcellulose of the capsule preferably is from plant origin.

This results in 21 mg of iron per capsule.

If the iron content of the Composition according to example 2 is deviating from the Standard Composition with 3.6 weight % of iron the amount of composition in the capsule can be adapted to result in 21 mg of iron per capsule.

### Example 4: Pharmaceutical Formulation 2

583 mg of Standard Composition (see Example 2) and 87 mg of acerola extract were filled into a standard pharmaceutical capsule (most often designed for 1 g of material) made from hydroxypropylmethylcellulose. The hydroxypropylmethylcellulose of the capsule preferably is from plant origin. The 87 mg of acerola extract (17 weight-% Vitamin C) is comprising 15 mg of Vitamin C.

This results in 21 mg of iron and 15 mg of Vitamin C per capsule.

If the iron content of the Composition according to example 2 is deviating from the Standard Composition with 3.6 weight % of iron the amount of composition in the capsule can be adapted to result in 21 mg of iron per capsule.

### Example 5: Treatment of acute iron deficiency conditions

2 to 5 capsules per day of either the capsule formulation according to example 3 or 4 were administered orally to a human being in need thereof being subject to acute iron deficiency conditions / anemia. Preferably the capsules are taken/applied unchewed with abundant liquid, preferably fasting or 1 to 2 hours before a meal. Further, when starting the treatment initially a lower amount of capsules should be taken or the capsules being taken less frequent, slowly increasing it to the suggested dose regimen mentioned above.

### Example 6: Use of the Composition as dietary supplement

If a person/human only has a higher need for iron being supplied, 1 capsule per day of either the capsule formulation according to example 3 or 4 should be administered orally Preferably the capsules are taken/applied unchewed with abundant liquid, preferably fasting or 1 to 2 hours before a meal. Further, when starting the treatment initially a lower amount of capsules should be taken or the capsules being taken less frequent, slowly increasing it to the suggested dose regimen mentioned above.

Preferably the formulation should not contain colorants, nor preserving agents, nor gluten, nor lactose and should also be useable by diabetics.

### Example 7: Test of Uptake and Tolerability in Volunteers

The composition according to example 2 of the invention was tested for the bioavailability (uptake) and tolerability compared to iron salts, e.g. to iron gluconate, after oral application to female volunteers.

In 2 studies an iron dietary supplement, iron(II) gluconate with ascorbic acid (known from the art) was compared to compositions with iron complexes according to the invention (example 2) regarding the concentration of iron in the serum of women receiving either the known iron supplement or the composition according to example 2 orally.

### Study 1): Iron(II) gluconate with ascorbic acid

Five women showing a latent iron deficiency were treated with 90 mg of iron in the form of iron(II) gluconate with vitamin C (900 mg; with vitamin C serving to enhance uptake) and the iron content in serum determined:
→ (Co) (Control; before taking the iron)
→ (2h) (2 hours after taking the iron)
→ (4h) (4 hours after taking the iron)

The results are shown in Fig 1) with A) being absolute and B) being relative data.

As an average the tests showed an absolute iron content increase of 42,6 µg/dl serum at 2 h and 52,4 µg/dl serum at 4 h after intake, meaning a relative increase by 44,7 or 54,1 % respectively.

Still, the increase in the iron concentration was accompanied by nausea or feeling of pressure in the abdomen; one of the women was even forced to vomit.

### Study 2): Composition according to the invention

Six women showing a latent iron deficiency were treated with a composition according to the invention according to example 2 leading to 90 mg of iron being applied per patient and the iron content in serum was determined:
→ (Co) (Control; before taking the iron)
→ (2h) (2 hours after taking the iron)
→ (4h) (4 hours after taking the iron)

The results are shown in Fig 2 with A) showing absolute and B) being relative data.

2 hours after the intake of the composition according to the invention (90 mg of iron) in average an absolute iron content increase in the blood by 61,3 µg/dl (2h), or after 4h to 70,7 µg/dl was measured.

This results in average to a relative increase in iron content of 70,9 or 83,52 % respectively owned to the higher bioavailability of the iron from plant origin. The probands did not show any side effect, proving a high degree of tolerability.

In fig. 3 and 4 the relative results of iron increase of both studies shown above are directly compared showing the superiority of the composition according to the invention.

### Example 8: Additional Test on Resorption and Tolerability

### Bioavailability of iron from the composition according to the invention in humans

The bioavailability of iron from the composition according to the invention is compared to an iron-salt preparation in healthy human individuals. Probands receive a single dose of iron from the composition according to the invention or iron-sulfate (double-blinded). Levels of serum iron are measured before and 15/30/60/120/240 minutes after the application of iron.

## Claims

1. Composition extracted from the leaves of the curry tree (*murraya koenigii*) comprising at least 1 weight % of iron.

2. Composition according to claim 1 comprising at least 2 weight % of iron, preferably at least 3 weight % of iron, more preferably at least 3.5 weight % of iron.

3. Composition according to claim 1 comprising 1 to 8 weight % of iron, preferably 2 to 7 weight %, more preferably 3 to 6 weight %, most preferably 3.5 to 5.5 weight % of iron.

4. Composition according to any of claims 1 to 4 obtainable by a process comprising the following steps:
• Step (i): Optionally milling or grinding of the leaves of *murraya koenigii* to achieve a powder of the leaves before the extraction step (ii);
• Step (ii): Extracting leaves of *murraya koenigii* at least once with an aqueous solution;
• Step (iii): Optionally, filter the extract from step (ii);
• Step (iv): Optionally, at least partly remove solvent from the extract from step (ii) or (iii);
• Step (v): Remove solvent from the extract from step (ii), (iii) or (iv) until dryness;
• Step (vi): Powdering the dry product of step (v);
• Step (vii): Optionally sieving the powdered product of step (vi);

5. Composition according to claim 4, in which in the steps of the process:
• Before step (i) the leaves of *murraya koenigii* are optionally pretreated by one or more optional operation such as washing, cleaning, soaking, drying, cutting, chopping, and/or blanching; and/or
• In step (i), the milling and grinding of the leaves of *murraya koenigii* to achieve a powder of the leaves before the extraction step (ii) is done with a mechanical device; and/or
• In step (ii):
➢ the extraction of the leaves of *murraya koenigii* is done with a solution comprising more than 50%(v/v) of water, preferably is water; and/or
➢ the extraction of the leaves of *murraya koenigii* is repeated at least once, preferably is repeated once, twice or thrice; and/or
➢ the extraction of the leaves of *murraya koenigii* is done at ambient temperature or above, preferably is done above 50°C, more preferably is done between 60 and 90°C, most preferably between 70 and 80°C.

6. Process for the production of a composition according to any one of claims 1 to 5 comprising the following steps:
• Step (i): Optionally milling or grinding of the leaves of *murraya koenigii* to achieve a powder of the leaves before the extraction step (ii);
• Step (ii): Extracting leaves of *murraya koenigii* at least once with an aqueous solution;
• Step (iii): Optionally, filter the extract from step (ii);
• Step (iv): Optionally, at least partly remove solvent from the extract from step (ii) or (iii);
• Step (v): Remove solvent from the extract from step (ii), (iii) or (iv) until dryness;
• Step (vi): Powdering the dry product of step (v);
• Step (vii): Optionally sieving the powdered product of step (vi).

7. Process according to claim 6, wherein.
• Before step (i) the leaves of *murraya koenigii* are optionally pretreated by one or more optional operation such as washing, cleaning, soaking, drying, cutting, chopping, and/or blanching; and/or
• In step (i), the milling and grinding of the leaves of *murraya koenigii* to achieve a powder of the leaves before the extraction step (ii) is done with a mechanical device; and/or
• In step (ii):
➢ the extraction of the leaves of *murraya koenigii* is done with a solution comprising more than 50%(v/v) of water, preferably is water; and/or
➢ the extraction of the leaves of *murraya koenigii* is repeated at least once, preferably is repeated once, twice or thrice; and/or
➢ the extraction of the leaves of *murraya koenigii* is done at ambient temperature or above, preferably is done above 50°C, more preferably is done between 60 and 90°C, most preferably between 70 and 80°C.

8. Medicament comprising a composition according to any of claims 1 to 5 and optionally one or more pharmaceutically acceptable excipients.

9. Medicament according to claim 8, wherein the iron comprised therein fully stems from plant sources, preferably from the composition according claim 1.

10. Medicament according to any of claims 8 or 9, further comprising Vitamin C.

11. Medicament according to claim 10 comprising 1 to 8 weight-% of iron and optionally 2 to 5 weight-% of Vitamin C.

12. Medicament according to any of claims 10 or 11, wherein the Vitamin C comprised therein fully stems from plant sources, preferably from acerola (*Malpighia glabra*) fruits.

13. Use of a composition according to any one of claims 1 to 5 for the production of a medicament for the treatment of iron deficiency, iron deficiency conditions, anemia, or iron deficiency anemia.

14. Composition according to any of claim 1 to 5 for use as a dietary supplement.

15. Composition according to any of claim 1 to 5 for use in supplementing an increased need for iron during pregnancy or lactation, during adolescence or competitive sports, following strong blood loss after surgery, gastrointestinal bleedings or strong menstruation; or during impairment of digestion or absorption of iron or diarrhea, or gastro-intestinal disease.
